# EUROPEAN PATENT APPLICATION

(11) **EP 0 541 251 A1**
(43) Date of publication of application: **12.05.1993**
(21) Application number: 92309209.2
(22) Date of filing: 09.10.1992
(51) Int. Cl.: A61F 13/02

(54) **A flexible wound dressing**

(30) Priority: 01.11.1991 US 786377
(71) Applicant: FERRIS MFG., CORP., Burr Ridge, Illinois 60521 (US)
(72) Inventor: Sessions, Robert W., Hinsdale, Illinois 60521 (US); Carr, Roy D., Burr Ridge, Illinois 60521 (US); Schmeichel, Rainer, Glen Ellyn, Illinois 60137 (US)
(74) Representative: Knott, Stephen Gilbert

(57) **Abstract**

The dressing (10) comprises a pad which contacts the wound without adhering thereto and absorbs wound exudate, a substrate (14) adhered to the pad which is impermeable to both bacteria and liquid water, the substrate allowing the extent of wound exudate absorption by the pad to be observed therethrough, and a cover sheet (16) adhered to the substrate and having an opening therethrough, the substrate and pad being dimensioned such that they extend beyond the dimensions of the cover opening while allowing a portion of adhesive on the cover sheet to remain uncovered to permit the dressing to be adhered to a patient's skin around the wound and so no adhesive contacts the wound. A method for the application of the aforedescribed dressing is also contemplated which comprises applying the dressing onto the wound.

## Description

The present invention relates to wound dressings generally and, more particularly, to dressings which are commonly referred to as window dressings.

Historically, the treatment of wounds consisted of "packing" or applying sterile dressings, typically gauze and/or lint, onto the injured area. The absorptive properties of these materials is beneficial to the healing process as they assist in the removal of inflammatory wound exudate and particles. These particles, if left in place, would hinder tissue repair or cause eschar formation about the wound. Moreover, some necrotic debris and bacteria are likewise removed by the use of such absorbent materials as autolysis, i.e., chemical debridement, is stimulated.

However, when these dressing materials become saturated or otherwise contaminated such that a change of dressings is indicated, several disadvantages are observed. Upon removal of the dressing, the dressing materials typically adhere to the tissue being treated and/or leave fibers in the wound. Further, upon removal of the old dressing, portions of the newly regenerated tissue remain on the dressing itself, creating additional trauma. While newer materials have attenuated these problems to a certain degree, they remain a serious concern.

Complicating the problems noted previously is the difficulty in determining precisely when a given dressing should be changed. The method typically used to determine when a dressing should be changed is by removal of the dressing from the wound and conducting a visual observation of the wound situs and the dressing itself. While the prudent application of fresh dressings is beneficial to the healing process, it is both economically inefficient and, as noted previously, harmful to the healing process, to change the dressings excessively. In addition, other problems arise when the dressing is continually removed to determine whether a fresh dressing is required. For example, each time the dressing is removed, the adhesive which keeps the dressing over the wound becomes less effective, ultimately resulting in adhesive failure. In addition, each removal of the dressing results in a disturbance of the existing, and desirable, sterile environment about the wound. This disturbance allows bacteria and other microbes to enter the body via the wound, thereby prolonging recovery.

In view of the foregoing, there exists a need for a wound dressing which provides a means for effectively determining the optimal time to change the dressing while minimizing any adverse effect on the healing process. Additionally, the dressing should allow, to the extent possible, a sterile environment to be maintained in the wound situs while allowing a degree of breathability above, and therefore to, the wound site. Moreover, the dressing should provide a means by which its adhesive capabilities are maintained until such time as a new dressing is required. Further, the dressing should provide for the absorbance of wound exudate. In addition, all of the foregoing should be present in a dressing which provides advantages in handling and delivery as well as from an economic perspective.

It is therefore an object of the present invention to provide a simple, yet effective means for visually determining the extent of wound exudate absorption by a dressing while minimizing the potential for bacterial infection of the wound.

A related object is to provide a means by which the optimal time for changing of the dressing may be determined while minimizing disturbance to the newly formed replacement tissue.

Another related object of the present invention is to minimize the dressing costs for treating a particular wound.

Another object of the present invention is to assist in the removal of liquid exudate from the wound situs thereby enhancing the healing process.

A further object of the present invention is to maintain the adhesive properties of the dressing until such time as a fresh dressing is required.

Yet another object of the present invention is to provide a dressing that is capable of providing a degree of breathability over the wound situs while minimizing the potential for bacterial contamination of the wound.

Another object of the present invention is to provide a dressing that is relatively simple to handle and deliver.

These an other objects and advantages of the present invention will become apparent from the following description of the invention.

The present invention provides a flexible wound dressing and related method for the treatment of wounds. More particularly, the dressing comprises a pad which contacts the wound without adhering thereto and absorbs wound exudate. A substrate may also be incorporated into the dressing and is adhered to the pad. This substrate is impermeable to both bacteria and liquid water and also allows the extent of wound exudate absorption by the pad to be observed through the substrate. A cover sheet, which has an opening therethrough, is also adhered to the substrate. Moreover, the size of the pad and substrate are dimensioned such that they extend beyond the dimensions of the cover opening while allowing a portion of adhesive on the cover sheet to remain uncovered to permit the dressing to be adhered to a patient's skin around the wound. In this way, no adhesive contacts the wound.

A method for the application of the aforedescribed dressing is also contemplated which comprises applying the dressing onto the wound.

The present invention may be better understood by reference to the following drawings and detailed description.
FIGURE 1 illustrates a perspective view of an embodiment of the present invention;
FIG. 2 is another perspective view of an alternative embodiment of the present invention; and
FIG. 3 is an exploded, cross-sectional view taken along line 3----3 of FIG. 2.

While the invention will be discussed in connection with certain preferred embodiments, it is not intended to be so limited. On the contrary, the invention is intended to cover all alternatives, modifications, and equivalents as may be included within the spirit and scope of the invention as defined by the appended claims.

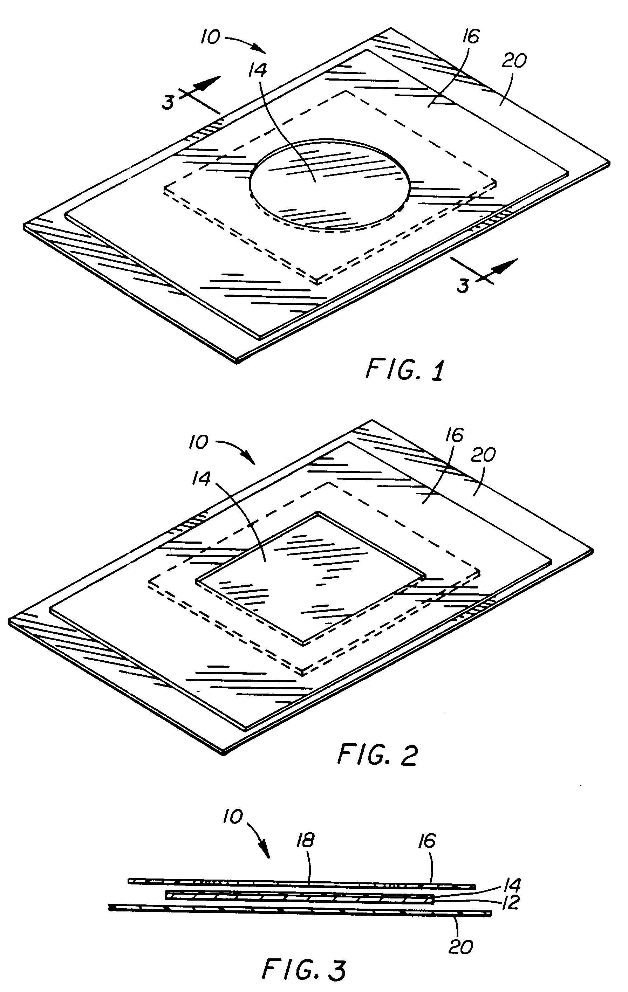

Referring to the figures, FIG. 1 illustrates an embodiment of the invention designated by the numeral 10. This embodiment comprises a pad 20, a substrate 14 which is adhered to the pad, a cover sheet 16 having an opening therethrough, and optionally, a release sheet 20. In this embodiment, a medical dressing is shown which is useful for the treatment of wounds. Turning to the individual components of this dressing, a pad 12 is provided which is designed to be placed an contact with a wound such that absorption of wound exudate occurs. As such, the pad should advantageously extend at least over the entire wound surface and preferably beyond same, to maximize the amount of exudate absorbed by a given dressing.

While this pad can be produced from any absorptive material, it should be produced from a material that will not adhere to the wound itself. Without such non-adherence, portions of newly regenerated tissue would remain on the dressing itself, and portions of the dressing in the wound, creating additional trauma to the wound. While non-adherent cloth may be used, a polymer-based foam is the preferred pad material in view of its superior properties versus cloth in the areas of liquid absorptivity and air and liquid-vapor permeability which assists in the removal of liquid from the wound situs. Most preferred is the polyurethane foam disclosed in U.S. Patent Application No. 175,036, filed March 29, 1988, to Sessions et al. entitled "Hydrophilic Foam Compositions", the content of this application being incorporated herein by reference.

A further characteristic of the pad material is that it allows a caregiver, upon viewing the side of the pad that is not in contact with the wound, to determine whether the dressing should be changed. In this respect, the pad should have the ability of changing its appearance when it absorbs liquid, such as wound exudate. Any visibly perceptible change is sufficient, for example, the pad could darken or discolor upon absorption of fluid. Thus, the invention allows for the successful use of an opaque pad, i.e., wherein the actual wound cannot be seen through the pad material, so long as the pad changes visually to a degree that is sufficient to permit an observer to ascertain that liquid absorption has occurred. This aspect of the invention is important in that it provides a simple, yet effective means for determining the degree of wound exudate adsorption of a dressing while not requiring the dressing to be removed from the wound.

The present invention may further comprise a flexible substrate 14, a first side of which is adhered to a second side of the pad, i.e., to the side of the pad that is not in contact with the wound. Although the substrate may be transparent, and is advantageously so, it need only allow the degree of wound exudate absorption by the pad to be observed therethrough. As such, any substrate material that would allow one to observe the discoloring of the pad material can be used.

The substrate material itself is impermeable to both bacteria and liquid water. This is particularly important in that many available pad materials are not impermeable to bacteria. As such, one function of the substrate is to prevent contamination of the wound situs by bacteria which, if the pad were used alone, would pass from the environment, through the pad, and onto the wound situs.

Advantageously, the substrate is also permeable to air and liquid vapors. This property allows liquid exudate in the form of vapor to be removed from the wound situs, which assists in the healing process. The substrate should possess a MVTR transmission rate of from 100 to 4000 g/m²/24 hrs and, preferably, 200 to 1000 g/m²/24 hrs. Such transmission rates are sufficient to provide an adequate rate of liquid vapor removal without allowing the wound to dry excessively. Examples of suitable gas and liquid vapor permeable substrate materials include polyurethane film and copolyester film.

The interrelationship between the substrate and pad materials is also important from the vapor transmission rate perspective. When both materials are gas and liquid-vapor permeable, try pad and substrate combination should have a MVTR of from 100 to 4000 g/m²/24 hrs, and preferably from 200 to 1000 g/m²/24 hrs. This is significant especially when a foam pad is utilized, such as urethane foam, which may have a MVTR as high as 5000 g/m²/24 hrs which would allow the wound to dry excessively, disturbing the healing process. As such, the composition of the substrate is important as the substrate will serve to limit the overall MVTR of the dressing within the desired parameters in cases where pad materials having undesirably high MVTR values are used.

While the substrate is adhered to one side of the pad, it need not be adhered by an adhesive per se in all cases. If, as in a preferred embodiment, the pad material is a foam, the foam precursor material may be applied onto the substrate during the production of the foam. In such a case, the substrate acts as a liner for the foam precursor and, upon rising and curing, the foam adheres thereto.

The dressing further includes a cover sheet 16 having an opening 18 therethrough which allows the substrate, and the pad, to be viewed. The cover is preferably produced by die-cutting the perimeter of the cover and the opening simultaneously from a sheet of cover material. An adhesive is disposed on an underside of the cover such that the second side of the substrate, i.e., that which is not adhered to the pad, is adhered to the cover. However, the substrate, as well as the pad, are preferably sized so that they extend beyond the dimensions of the cover opening while allowing the adhesive around the perimeter of the underside of the cover sheet to remain uncovered by the substrate or pad. In this way, the cover sheet may be adhered to a patient's skin around the wound without any adhesive contacting the wound. This also has the advantage of enabling the wound to be sealed off from the atmosphere, thereby allowing the sterility of the wound environment to be maintained. In the alternative, the substrate and pad may be sized differently so that only a portion of the cover is adhered to the skin. For example, the substrate and pad may be sized so that only the ends of the cover adhere to the skin.

While the cover can be produced from almost any material, a material that is gas and liquid-vapor permeable is preferred. Such a material acts to prevent maceration of the tissue to which the dressing is adhered while the dressing is in place over the wound. Flexibility in the cover material is also advantageous as this allows the dressing to be successfully applied onto non-planar areas of the body. Examples of such permeable materials include polyvinyl chloride foam and urethane foam.

The flexible dressing of the present invention provides several advantages. In one aspect, the present invention further minimizes the potential for bacterial infection of the wound because the need for changing the bandage can be determined without removal of the dressing. Healing is also enhanced by the absorptivity and breathability of the dressing. Moreover, exposure of the wound to the atmosphere which would typically occur when prior art dressings were lifted from the wound to determine the degree of absorption is now not required. In addition, such lifting has the effect of reducing the adherence of the adhesive which holds the dressing onto the body--each time the dressing is lifted and replaced, body oils and fluids can contaminate the adhesive, rendering it less adherent. Such lack of adherence would disturb the sterile environment created by the dressing, potentially resulting in wound contamination. The present invention also provides a solution to this problem. Further, the dressing, while flexible enough to allow the successful delivery of the dressing onto non-planar wound areas, is sufficiently inflexible such that handling and delivery problems are minimized, e.g., the dressing does not have the tendency to readily fold over upon itself.

A release sheet 20 may also be advantageously provided for covering the adhesive residing on the underside of the cover until such time as the dressing is ready for use. The inclusion of this sheet improves the handling characteristics of the dressing by maintaining the adhesive unexposed. At such time, the release liner may be peeled off, exposing the adhesive, the dressing centered over the wound, and applied thereto.

In view of the foregoing, the present invention provides a wound dressing having a means for effectively determining the optimal time to change the dressing while maintaining optimal conditions for the healing process, e.g., removal of the wound exudate, minimizing disturbance to the newly formed replacement tissue and minimizing the potential for bacterial contamination of the wound. In addition, the dressing allows a sterile environment to be maintained in the wound situs while the determination of the dressing's condition is being made. Moreover, the present invention provides a dressing that is relatively easy to handle and deliver. The dressing is also capable, due to its design which does away with frequent and premature lifting of the dressing to visually inspect the wound, to maintain its adhesive capabilities until such tine as a new dressing is required. Further, the dressing absorbs wound exudate while providing breathability over the wound situs, enhancing the healing process.

## Claims

1. A flexible dressing for application onto a wound comprising
a pad which contacts the wound without adhering thereto and absorbs wound exudate,
a substrate adhered to the pad which is impermeable to bacteria, the substrate allowing the extent of wound exudate absorption by the pad to be observed therethrough, and
a cover sheet adhered to the substrate and having an opening therethrough,
the substrate and pad being dimensioned such that they extend beyond the dimensions of the cover opening while allowing a portion of adhesive on the cover sheet to remain uncovered to permit the dressing to be adhered to a patient's skin around the wound and so no adhesive contacts the wound.

2. The wound dressing according to claim 1, wherein the pad and substrate are permeable to air and liquid vapor.

3. The wound dressing according to claim 2, wherein the MVTR of the substrate ranges from 100 to 4000 g/m²/24 hrs.

4. The wound dressing according to claim 3, wherein the MVTR of the substrate and pad combination range from 100 to 4000 g/m²/24 hrs.

5. The wound dressing according to claim 4, wherein said substrate is transparent and is selected from the group consisting of polyurethane film and copolyester film.

6. The wound dressing according to claim 5, wherein said cover material is selected from the group consisting of polyvinylchloride foam and urethane foam.

7. The wound dressing according to claim 1, wherein the dimensions of the pad are such that the area of the pad is equivalent to the area of the substrate, the pad extending over the entire wound.

8. The wound dressing according to claim 1, wherein said substrate and said pad are dimensioned such that they extend beyond the dimensions of the cover opening while allowing adhesive around the perimeter of the cover sheet to remain uncovered such that when the dressing is adhered to a patient's skin the dressing seals the wound from the environment.

9. A flexible dressing for application onto a wound comprising
a polyurethane foam pad which is air and liquid-vapor permeable which contacts the wound without adhering thereto and absorbs wound exudate,
a transparent substrate adhered to the pad which is air and liquid-vapor permeable but impermeable to bacteria, the substrate allowing the extent of wound exudate absorption by the pad to be observed therethrough, and
a urethane foam cover sheet having an opening therethrough and an adhesive disposed on an underside thereof, wherein the substrate is adhered to the underside of the cover sheet,
the substrate and pad being equivalent in area and further dimensioned such that they extend beyond the dimensions of the cover opening while allowing the adhesive around the perimeter of the underside of the cover sheet to remain uncovered such that when the sheet is adhered to a patient's skin around the wound, no adhesive contacts the wound and the wound is sealed from the environment,
the combination of the substrate and pad having a MVTR ranging from 100 to 4000 g/m²/24 hrs.

10. A flexible dressing for application onto a wound comprising
a pad which contacts the wound without adhering thereto and absorbs wound exudate and
a cover sheet having an opening therethrough which opening allows the extent of wound exudate absorption by the pad to be observed therethrough,
the pad being dimensioned such that it extends beyond the dimensions of the cover opening while allowing a portion of adhesive on the cover sheet to remain uncovered to permit the dressing to be adhered to a patient's skin around the wound and so no adhesive contacts the wound.

11. The wound dressing according to claim 10, wherein the pad is permeable to air and liquid vapor and the MVTR of the padranges from 100 to 4000 g/m²/24 hrs.

12. The wound dressing according to claim 11, wherein the dimensions of the pad are such the pad extends over the entire wound.

13. The wound dressing according to claim 12, wherein said pad is dimensioned such that it extends beyond the dimensions of the cover opening while allowing adhesive around the perimeter of the cover sheet to remain uncovered such that when the dressing is adhered to a patient's skin the dressing seals the wound from the environment.

14. A method for the treatment of wounds comprising applying a flexible dressing onto the wound, said dressing comprising
a pad which contacts the wound without adhering thereto and absorbs wound exudate,
a substrate adhered to the pad which is impermeable to bacteria, the substrate allowing the extent of wound exudate absorption by the pad to be observed therethrough, and
a cover sheet adhered to the substrate and having an opening therethrough,
the substrate and pad being dimensioned such that they extend beyond the dimensions of the cover opening while allowing a portion of adhesive on the cover sheet to remain uncovered to permit the dressing to be adhered to a patient's skin around the wound and so no adhesive contacts the wound.

15. The method according to claim 14, wherein the pad and substrate are permeable to air and liquid vapor.

16. The method according to claim 15, where in the MVTR of the substrate and pad combination ranges from 100 to 4000 g/m²/24 hrs.
